(19) Europäisches Patentamt
European Patent Office
Office européen des brevets

(11) Publication number: 0 034 752
B1

(12) # EUROPEAN PATENT SPECIFICATION

(45) Date of publication of patent specification: 08.06.83

(21) Application number: 81100929.9

(22) Date of filing: 10.02.81

(51) Int. Cl.³: C 07 D 295/14, C 07 D 211/18, C 07 D 211/14, A 61 K 31/495, A 61 K 31/445

(54) **Phenoxyalkane derivative and processes for preparing same.**

(30) Priority: 22.02.80 JP 21928/80
22.02.80 JP 21929/80
22.02.80 JP 21930/80

(43) Date of publication of application:
02.09.81 Bulletin 81/35

(45) Publication of the grant of the patent:
08.06.83 Bulletin 83/23

(84) Designated Contracting States:
DE FR GB

(56) References cited:
GB - A - 2 007 655
US - A - 3 822 266

(73) Proprietor: Tanabe Seiyaku Co., Ltd.
No. 21 Dosho-machi 3-chome Higashi-ku
Osaka-shi Osaka-fu (JP)

(72) Inventor: Kanno, Takeshi
No. 477-12 (Konba-cho-danchi 12-302) Konba-cho
Omiya-shi Saitama-ken (JP)
Inventor: Gaino, Mitsunori
RC-5-306 Sunadanchi No. 4-1, Higashi-Omiya 3-chome
Omiya-shi Saitama-ken (JP)
Inventor: Yamamura, Michio
No. 878, Oaza-Kata
Tondabayashi-shi Osaka-fu (JP)
Inventor: Ishida, Ryuichi
No. 19-6, Yamada-Nishi 1-chome
Suita-shi Osaka-fu (JP)
Inventor: Shintomi, Keiichi
No. 3-C10-301, Aoyamadai 4-chome
Suita-shi Osaka-fu (JP)

(74) Representative: Kraus, Walter, Dr. et al,
Patentanwälte Dres. Kraus & Weisert
Irmgardstrasse 15
D-8000 München 71 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 034 752 B1

# Phenoxyalkane derivative and processes for preparing same

This invention relates to a phenoxyalkane derivative and processes for preparing same. More particularly, it relates to a phenoxyalkane derivative of the formula:

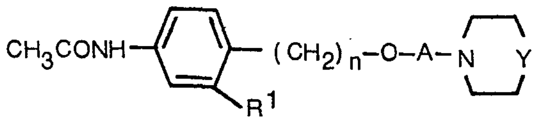 (I)

wherein $R^1$ is methoxy, n is zero, A is alkylene of 2 to 4 carbon atoms and Y is a group of the formula:

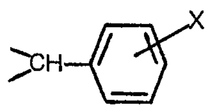 or 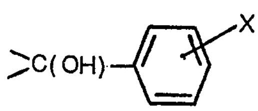

wherein X is hydrogen or halogen; or

$R^1$ is hydrogen or methoxy, n is an integer of 1, A is alkylene of 2 to 3 carbon atoms and Y is a group of the formula:

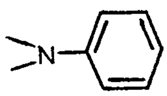 or

$R^1$ is methoxy, n is zero, Y is a group of the formula:

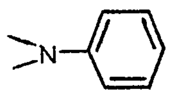 ;

and A is a branched alkylene of the formula:

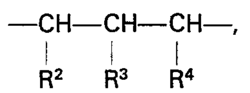

wherein either one of $R^2$, $R^3$ and $R^4$ is methyl and the remaining two groups of $R^2$, $R^3$ and $R^4$ are hydrogen,
or a pharmaceutically acceptable acid addition salt thereof.

Cerebral edema, which is a well-known complication of various cerebral diseases (e.g., cerebral hemorrhage, subarachnoid hemorrhage, cerebral thrombosis, cerebral embolism, head injury, cerebral tumor and encephalomyelitis), induces an increase in intracranial pressure due to compression of neighbouring brain tissues. Moreover, such increased intracranial pressure is known to adversely affect cerebral metabolism, result in disturbances of cerebral circulation and further aggravate cerebral edema. Therefore, increased intracranial pressure exerts serious damaging effects on patients or is sometimes fatal to them.

GB—A—2 007 655 discloses that 1-substituted-4-aroyl-4-hydroxy-piperidines such as 1-[3-(p-acetyl-o-methoxy-phenoxy)propyl]-4-(p-fluorobenzoyl)-4-hydroxy-piperidine have central nervous system depressing properties. However, this prior art reference does not disclose or suggest the inventive phenoxy-alkane derivatives.

As a result of various investigations, we have now found that the phenoxyalkane derivative (I) of the present invention shows a significant decrease in intracranial pressure. For example, when a solution of a test compound in an aqueous 5 w/v% mannitol solution was infused via the femoral vein of rats at a rate of 0.2 ml/kg/minute for 20 minutes, 1-(4-acetamidobenzyloxy)-3-(4-phenylpiperazino)-n-propane (dose: 1 mg/kg) showed about 23% decrease in the intracranial pressure 40 minutes after administration of the test compound.

In view of the effect of the phenoxyalkane derivative (I) on intracranial pressure, said compound of the present invention is useful for treating a warm-blooded animal, including human, suffering from increased intracranial pressure due to various cerebral diseases such as cerebral infarction, cerebral thrombosis, cerebral embolism, cerebral hemorrhage, subarachnoid hemorrhage, head injury, cerebral tumor, cerebral edema, encephalomyelitis and the like.

Moreover, since the phenoxyalkane derivative (I) of the present invention shows a potent

depressing effect of central nervous system, said compound is also useful as a tranquilizer, an analgesic or an anti-vomitting agent.

Examples of the phenoxyalkane derivative include those of the formula (I) in which $R^1$ is methoxy, n is zero, A is alkylene such as ethylene, trimethylene or tetramethylene, Y is a group of the formula:

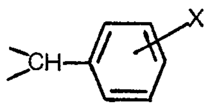 or 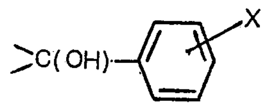 ,

and X is hydrogen or halogen such as chlorine; or $R^1$ is hydrogen or methoxy, n is an integer of 1, A is alkylene such as ethylene or trimethylene, and Y is a group of the formula:

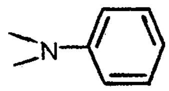 ;

or $R^1$ is methoxy, n is zero, A is branched alkylene such as a group of the formula:

$$-CH_2CH(CH_3)CH_2-,\ -CH(CH_3)-CH_2CH_2-\ \text{or}\ -CH_2CH_2CH(CH_3)-,$$

and Y is a group of the formula:

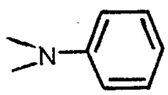 .

Among them, a preferred subgenus includes the compound of the formula (I) in which $R^1$ is methoxy, n is zero, A is trimethylene and Y is a group of the formula:

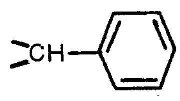 ;

or $R^1$ is hydrogen or methoxy, n is an integer of 1, A is trimethylene and Y is a group of the formula:

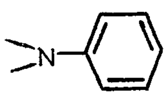 ;

or $R^1$ is methoxy, n is zero, A is a group of the formula:

$$-CH_2CH(CH_3)CH_2-\ \text{or}\ -CH_2CH_2CH(CH_3)-,$$

and Y is a group of the formula:

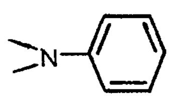 .

Further preferred subgenus includes the compound of the formula (I) in which $R^1$ is hydrogen or methoxy, n is an integer of 1, A is trimethylene and Y is a group of the formula:

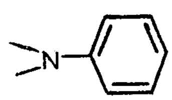 ;

or $R^1$ is methoxy, n is zero, A is a group of the formula:

$$-CH_2CH(CH_3)CH_2-,$$

and Y is a group of the formula:

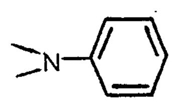 .

The compound (I) of the present invention can be used for pharmaceutical use either as the free

base or a pharmaceutically acceptable acid addition salt thereof. Pharmaceutically acceptable acid addition salts of the compound (I) include, for example, inorganic acid addition salts (e.g., hydrochloride, hydrobromide, sulfate, nitrate, phosphate) and organic acid additon salts (e.g., acetate, lactate, oxalate, citrate, tartrate, fumarate, maleate, methanesulfonate, benzoate). The compound (I) of the present invention can be administered either orally or parenterally. A daily dose of the compound (I) may be about 0.05 to 50 mg (in terms of free base), especially 0.1 to 10 mg (in terms of free base), per kilogram of body weight. Further, the compound (I) may be used in the form of a pharmaceutical preparation containing the same compound in conjunction or admixture with a pharmaceutical excipient suitable for enteral or parenteral administration. Suitable excipients include, for example, gelatin, lactose, glucose, sodium chloride, starch, magnesium stearate, talcum, vegetable oil and other known excipients. The pharmaceutical preparation may be in solid form such as powder, tablets or capsules; or in liquid form such as solutions, suspensions or emulsions. The compound (I) may also be used in the form of an injection for drip infusion.

According to the present invention, the compound (I) in which $R^1$ is methoxy, n is zero, A is alkylene of 2 to 4 carbon atoms and Y is a group of the formula:

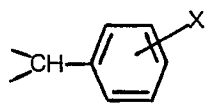 or 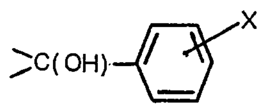

(X is the same as defined above); or $R^1$ is hydrogen or methoxy, n is an integer of 1, A is alkylene of 2 to 3 carbon atoms and Y is a group of the formula:

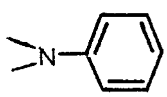

can be prepared by condensing a compound of the formula:

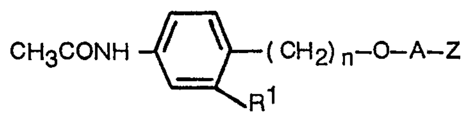 (II)

wherein Z is a reactive group or atom and $R^1$, A and n are the same as defined above, with the compound of the formula:

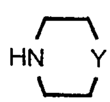 (III)

wherein Y is the same as defined above. On the other hand, the compound (I) in which $R^1$ is methoxy, n is zero, Y is a group of the formula:

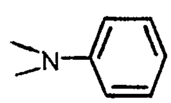 ,

and A is a branched alkylene of the formula:

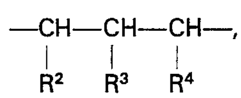

($R^2$, $R^3$ and $R^4$ are the same as defined above) can be prepared by condensing a compound of the formula:

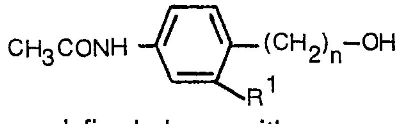 (IV)

wherein $R^1$ and n are the same as defined above, with a compound of the formula:

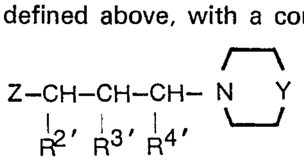 (V)

wherein either one of $R^{2'}$, $R^{3'}$ and $R^{4'}$ is methyl and the remaining two groups of $R^{2'}$, $R^{3'}$ and $R^{4'}$ are hydrogen, Z is a reactive group or atom and Y is the same as defined above.

Examples of the reactive group or atom (Z) in the starting compounds (II) and (V) include alkylsulfonyloxy (e.g., methylsulfonyloxy), arylsulfonyloxy (e.g., p-toluenesulfonyloxy) and halogen atom (e.g., chlorine, bromine).

The condensation reaction of the compounds (II) and (III) is readily accomplished in the presence or absence of an acid acceptor in a solvent. Alkanol (e.g., methanol, ethanol, propanol), dimethylformamide, dimethylsulfoxide and the like are suitable as the solvent. Examples of the acid acceptor include organic bases such as triethylamine, triethylenediamine or N-methylpiperidine; and inorganic bases such as potassium carbonate, sodium carbonate, potassium bicarbonate or sodium bicarbonate. It is preferred to carry out the reaction at a temperature of 20° to 100°C.

The condensation reaction of the compound (IV) and (V) is accomplished in the presence of an acid acceptor in a solvent. Alkanol (e.g., methanol, ethanol, propanol), dimethylformamide, dimethylsulfoxide and the like are suitable as the solvent. Examples of the acid acceptor include inorganic bases such as sodium hydroxide, potassium hydroxide, lithium hydroxide and the like. It is preferred to carry out the reaction at a temperature of 20° to 100°C. It is also preferred to carry it out in an inert gas (e.g., nitrogen gas) atmosphere. In carrying out this condensation reaction, a compound of the formula:

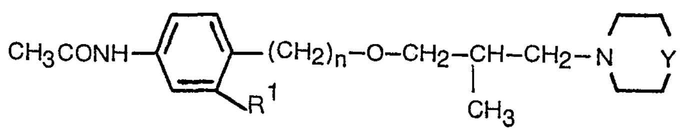

(I')

wherein $R^1$, n and Y are the same as defined above, is obtained by the use of a compound of the formula:

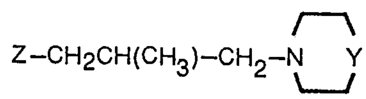

(V')

(wherein Z and Y are the same as defined above) as the starting compound. On the other hand, the condensation reaction of the compound (IV) with, for example, a compound of the formula:

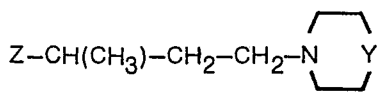

(V'')

gives the compounds of the formulae:

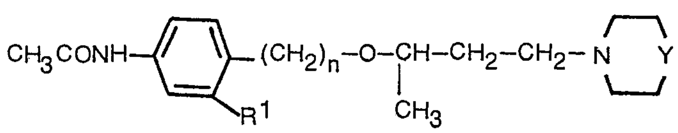

(I'')

and

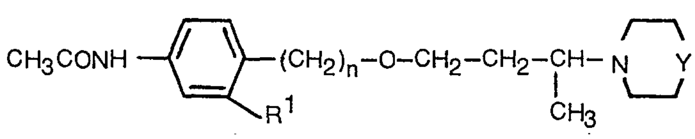

(I''')

wherein $R^1$, n and Y are the same as defined above. This may be probably due to a fact that the compound (V'') used as the starting compound reacts with the compound (IV) via a spiro compound of the formula:

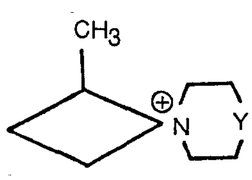

wherein Y is the same as defined above. These two compounds (I'') and (I''') thus obtained may be readily separated from one to another in a conventional manner, for example, by fractional recrystallization thereof.

The starting compounds of the present invention, i.e., the compounds (II) and (V), may be prepared by the methods described in the following reaction schemes.

*Synthesis of the compound (II)*

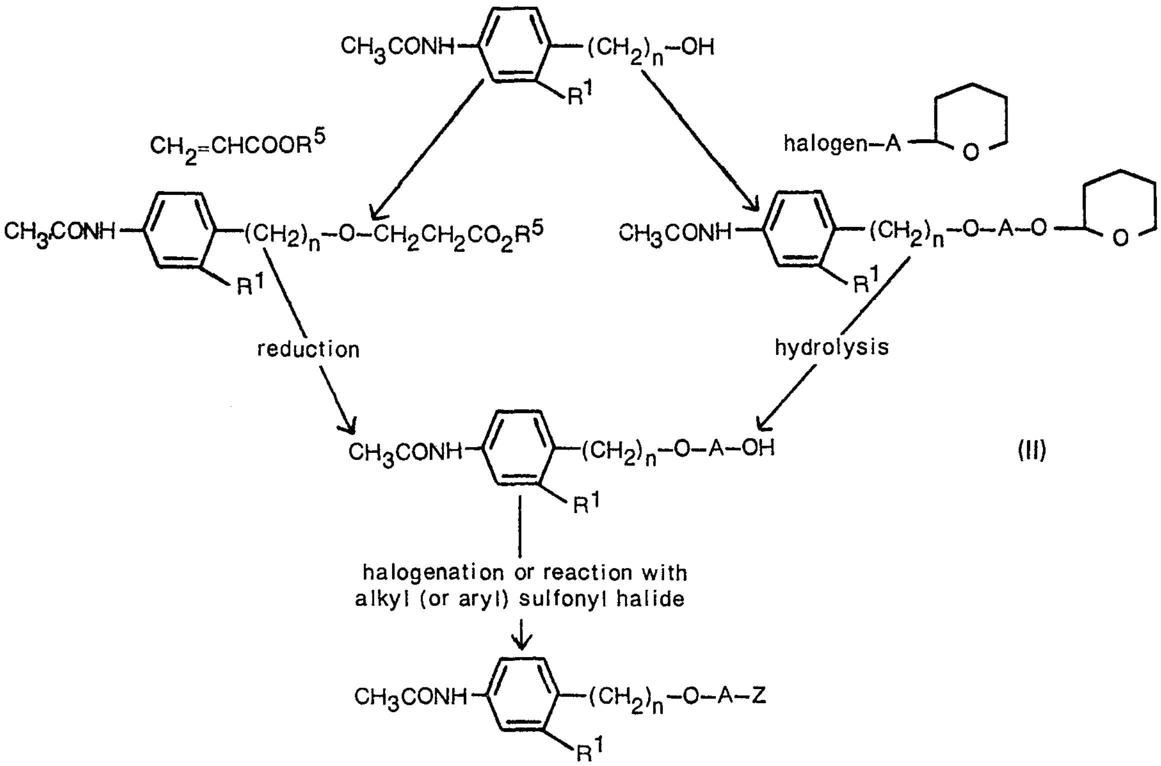


(II)

wherein $R^5$ is alkyl, and $R^1$, A, n and Z are the same as defined above.

*Synthesis of the compound (V)*

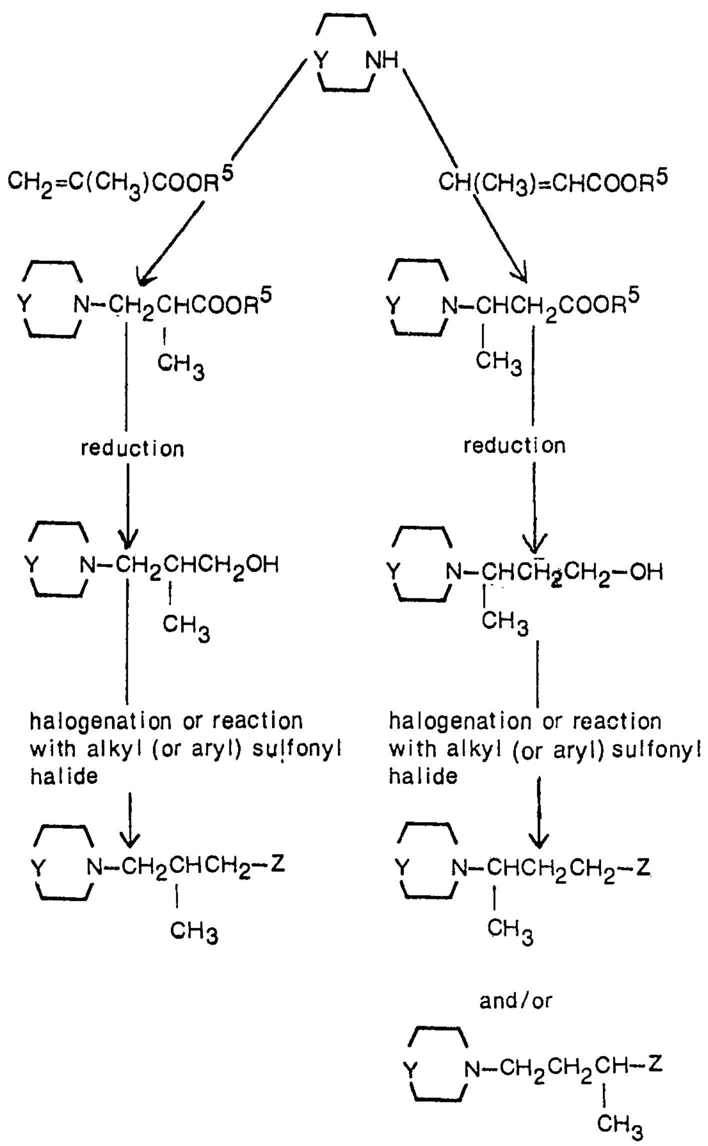


wherein Y, Z and $R^5$ are the same as defined above.

Experiment 1
(Effect of i.v., injection of test compounds on intracranial pressure in rats)

Male SD-rats weighing 500 to 800 g (each group consisting of 5 rats) were anesthetized with urethane. A solution of a test compound in an aqueous 5 w/v% mannitol solution was infused via the femoral vein at a rate of 0.2 ml/kg/minute for 20 minutes (when the test compound was used in the form of free base, said solution was prepared by dissolving it in an aqueous 5 w/v% mannitol solution with the air of 0.5 N hydrochloric acid). Intracranial pressure (i.e., cerebrospinal fluid pressure in the cisterna magna) was continuously monitored via a canula introduced in the cisterna magna. The canula was connected to a transducer, and recording were made on graph paper. The effect of the test compound on the intracranial pressure was estimated in terms of the increase or decrease in intracranial pressure, which was calculated in accordance with the following formula:

$$\text{Changes (\%) in intracranial pressure} = \left( \frac{\text{Intracranial pressure (mm } H_2O\text{) measured after administration of test compound}}{\text{Intracranial pressure (mm } H_2O\text{) measured before administration of test compound}} \right) \times 100$$

The results are shown in the following Table 1.

TABLE 1

| Test compounds (Dose: one mg/kg, i.v.) | Change (%) in intracranial pressure — Time after administration of Test compounds (minutes) 0* | 40 | 60 | 120 |
|---|---|---|---|---|
| 1-(4-acetamidobenzyloxy)-3-(4-phenylpiperazino)-n-propane | 100 | 77.0 | 83.1 | 97.9 |
| 1-(4-acetamido-2-methoxyphenoxy)-3-(4-phenylpiperazino)-2-methyl-n-propane | 100 | 66.4 | 73.8 | 83.8 |
| 1-(4-acetamido-2-methoxyphenoxy)-3-[4-(4-chlorophenyl)-4-hydroxy-piperidino]-n-propane | 100 | 94.2 | 95.4 | 90.7 |
| Control 5% mannitol solution (4 ml/kg, i.v.) | 100 | 106.4 | 107.5 | 114.4 |

Note: *: The intracranial pressure measured before administration of test compounds was 71.5±3.5 mm $H_2O$ (0 minute)

Experiment 2
(Effect of test compound on central nervous system in mice)

The effect of a test compound on central nervous system was examined by the following methods.

(1) Effect on spontaneous motor activity:

A solution or suspension of a test compound in water or an aqueous carboxymethylcellulose solution was administered intraperitoneally to male mice weighing 18 to 20 g (each group consisting of 5 mice). Thirty minutes after administration of the test compound, the spontaneous motor activity of the mice was measured with an animex activity meter for 5 minutes. The 50% effective dose ($ED_{50}$) of the test compound was estimated in terms of a dose which is necessary to decrease the motor activity of the medicated mice by 50% of that of a control group (i.e., non-medicated mice).

(2) Effect on sodium hexobarbital-induced anesthesia:

A solution or suspension of a test compound in water or an aqueous carboxymethylcellulose solution was administered intraperitoneally to male mice weighing 18 to 20 g (each group consisting of 5 mice). Thirty minutes after intraperitoneal administration of the test compound, sodium hexobarbital (100 mg/kg) was administered intraperitoneally to the mice, and the sleeping time was measured as the inverval between the loss and recovery of the righting reflex. The 50% effective dose ($ED_{50}$) of the test compound was estimated in terms of dose which is necessary to prolong the sleeping time of the medicated mice twice as long as that of a control group (i.e., non-medicated mice).

(3) Anti-apomorphine activity:

A solution or suspension of a test compound in water or an aqueous carboxymethylcellulose solution was administered intraperitoneally to male mice weighing 18 to 20 g (each group consisting of 5 mice). Thirty minutes after administration of the test compound, apomorphine hydrochloride (2.5

mg/kg) was administered subcutaneously to the mice, and the number of the mice which could climb a cage was examined. The 50% effective dose ($ED_{50}$) of the test compound was estimated in terms of a dose which is necessary to decrease the number of the medicated mice which could climb the cage by 50% of the number of a control group (i.e., non-medicated mice) which could climb the cage.

The results are shown in the following Table 2.

TABLE 2

| Test compound | MTD* | Effect on central nervous system ($ED_{50}$, mg/kg) | | |
|---|---|---|---|---|
| | | spontaneous motor activity | hexobarbital-induced anesthesia | Anti-apomorphine activity |
| 1-(4-acetamido-2-methoxybenzyloxy)-3-(4-phenyl-piperazino)-n-propane | 100 | 5 | 10 | 7 |

Note: *: Maximum tolerated dose (mg/kg, i.p.)

Example 1

(1) 13.4 g of 4-acetamido-2-methoxyphenol are dissolved in 80 ml of dimethylsulfoxide in nitrogen atmosphere, and 2.96 g of powdery sodium hydroxide are added thereto. The mixture is stirred in an oil bath (temperature: 50—55°C) for 3 hours. A solution of 10 g of 2-chloro-1-(tetrahydropyran-2-yl-oxy)ethane in 20 ml of dimethylsulfoxide is added dropwise to the reaction mixture, and said mixture is stirred at 100°C for one hour. After the reaction, the mixture is poured into ice-water and extracted with ethyl acetate. The extract is washed with water and an aqueous saturated sodium chloride solution, dried and then evaporated to remove solvent. The residue thus obtained is recrystallized from a mixture of ethyl acetate and n-hexane. 15.5 g of 1-(4-acetamido-2-methoxyphenoxy)-2-(tetrahydropyran-2-yl-oxy)ethane are obtained as colorless needles. Yield: 82.5% M.p. 88—90°C.

(2) 15.5 g of 1-(4-acetamido-2-methoxyphenoxy)-2-tetrahydropyran-2-yl-oxy)ethane are dissolved in a mixture of 70 ml of ethanol and 140 ml of water, and one ml of concentrated hydrochloric acid is added thereto. The mixture is stirred at room temperature for 2 days. Then, the reaction mixture is evaporated under reduced pressure to remove ethanol. After cooling the residue obtained, the crystalline precipitates are collected by filtration and recrystallized from ethanol. 9.43 g of 1-(4-acetamido-2-methoxyphenoxy)-2-hydroxy-ethane are obtained as colorless prisms. Yield: 84% M.p. 157—159°C.

(3) 5.0 g of 1-(4-acetamido-2-methoxyphenoxy)-2-hydroxy-ethane are dissolved in 120 ml of pyridine, and a solution of 5 g of p-toluenesulfonyl chloride in 30 ml of pyridine is added dropwise thereto at 0°—5°C. The mixture is allowed to stand for 5 days under ice-cooling. The reaction mixture is poured into ice-water and extracted with ethyl acetate. The extract is washed with 10% hydrochloric acid and water, dried and then evaporated under reduced pressure to remove solvent. The residue obtained is recrystallized from a mixture of ethyl acetate and n-hexane. 5.9 g of 1-(4-acetamido-2-methoxyphenoxy)-2-(p-toluenesulfonyloxy)ethane are obtained as colorless needles. Yield: 70% M.p. 106—107.5°C.

(4) 960 mg of 4-phenylpiperidine and 1.5 g of triethylamine are added to 10 ml of an anhydrous dimethylformamide solution containing 1.5 g of 1-(4-acetamide-2-metboxyphenoxy)-2-(p-toluenesulfonyloxy)ethane, and the mixture is stirred for 6 hours under heating in an oil bath (temperature: 100°C). After the reaction, the mixture is poured into ice-water and extracted with ethyl acetate. The extract is washed with water and an aqueous saturated sodium chloride solution. Then, the extract is dried and evaporated under reduced pressure to remove solvent. The residue thus obtained is recrystallized from benzene. 744 mg of 1-(4-acetamido-2-methoxyphenoxy)-2-(4-phenyl-piperidino)ethane are obtained as colorless needles. Yield: 51% M.p. 126—127°C.

$IR\nu_{max}^{nujol}$ ($cm^{-1}$): 3270, 3200, 1660, 1140, 1030

NMR ($CDCl_3$) $\delta$ : 7.89 (broad s, 1H, —N*H*—), 7.7—6.8 (m, 8H, aromatic), 4.10 (t, 2H, J=6.5 Hz, —O$CH_2CH_2$—), 3.75 (s, 3H, —O$CH_3$), 3.55—1.2 (m, 11H), 2.14 (s, 3H, $CH_3$CO—)

Mass m/e: 368 ($M^+$), 188, 174 (base peak)

Hydrochloride:

M.p. 165—166°C (colorless needles, recrystallized from isopropanol)

$IR\nu_{max}^{nujol}$ ($cm^{-1}$): 3200, 2700—2400, 1685, 1115, 1030

Example 2

(1-a) 5 ml of a 40% benzyltrimethylammonium hydroxide/methanol solution are added to a mixture of 6 g of 4-acetamido-2-methoxyphenol and 33 g of ethyl acrylate, and the mixture is stirred at 100°—110°C for 6 days. The reaction mixture is condensed under reduced pressure to remove excess ethyl acrylate. Chloroform is added to the residue, and the chloroform solution is washed with 2% hydrochloric acid, water and an aqueous saturated sodium chloride solution, successively. Then, the chloroform solution is dried and evaporated under reduced pressure to remove solvent. The residue is purified by silica gel chromatography (solvent: 2% methanol-chloroform). 1.47 g of 1-(4-acetamido-2-methoxyphenoxy)-2-(ethoxycarbonyl)ethane are obtained. M.p. 103—104°C (recrystallized from benzene).

(1-b) 250 mg of powdery sodium hydroxide are added to a mixture of 25 g of 4-acetamido-2-methoxyphenol and 138 g of ethyl acrylate, and the mixture is refluxed for 6 days. The reaction mixture is condensed under reduced pressure to remove excess ethyl acrylate. Chloroform is added to the residue, and the chloroform solution is washed with an aqueous saturated sodium chloride solution. Then, the chloroform solution is dried and evaporated under reduced pressure to remove solvent. The residue is purified by silica gel chromatography (solvent: 2% methanol-chloroform). 8.55 g of 1-(4-acetamido-2-methoxyphenoxy)-2-(ethoxy-carbonyl)ethane are obtained. M.p. 103—104°C (recrystallized from benzene).

(2) 5.55 g of lithium aluminium hydride are suspended in 500 ml of anhydrous tetrahydrofuran, and a solution of 21 g of 1-(4-acetamido-2-methoxyphenoxy)-2-(ethoxycarbonyl)ethane in 300 ml of anhydrous tetrahydrofuran is added dropwise thereto at a temperature below 0°C under stirring. The mixture is further stirred at the same temperature for 3 hours. Then, excess lithium aluminium hydride is decomposed, and insoluble materials are removed by filtration. The filtrate is condensed under reduced pressure to remove solvent. The residue obtained is recrystallized from a mixture of acetone and ethyl acetate. 14.47 g of 1-(4-acetamido-2-methoxyphenoxy)-3-hydroxy-n-propane are obtained as colorless needles. Yield: 81% M.p. 121—122°C.

(3) 14.47 g of 1-(4-acetamido-2-methoxyphenoxy)-3-hydroxy-n-propane, 17.5 g of p-toluenesulfonyl chloride and 130 ml of pyridine are treated in the same manner as described in Example 1-(3). The crude product thus obtained is recrystallized from benzene. 21.67 g of 1-(4-acetamido-2-methoxyphenoxy)-3-(p-toluenesulfonyloxy)-n-propane are obtained as colorless prisms. Yield: 91% M.p. 90—91°C.

(4) 693 mg of 4-phenylpiperidine and 400 mg of triethylamine are added to 40 ml of an ethanol solution containing 1.5 g of 1-(4-acetamido-2-methoxyphenoxy)-3-(p-toluenesulfonyloxy)-n-propane, and the mixture is refluxed for 26 hours. After the reaction, the mixture is evaporated under reduced pressure to remove ethanol. An aqueous saturated sodium hydroxide solution cooled with ice is added to the residue to adjust a pH of the mixture to 11, and said mixture is extracted with chloroform. The extract is washed with water and an aqueous saturated sodium chloride solution. Then, the extract is dried and evaporated under reduced pressure to remove solvent. The residue thus obtained is purified by silica gel chromatography (solvent: 5% methanol-chloroform) and then recrystallized from ethanol. 990 mg of 1-(4-acetamido-2-methoxyphenoxy)-3-(4-phenylpiperidino)-n-propane are obtained as colorless needles. Yield: 66% M.p. 149—150°C (decomp.).

IR$\nu_{max}^{nujol}$ ($cm^{-1}$): 3250, 1655, 1140, 1040

NMR ($CDCl_3$) $\delta$: 7.81 (broad s, 1H, —*NH*—), 7.25 (broad s, 5H, aromatic), 6.87 (broad s, 3H, aromatic), 4.05 (6, 2H, J=6.5 Hz, —$OCH_2CH_2$—), 3.80 (s, 3H, —$OCH_3$), 3.25—2.85 (m, 2H), 2.7—2.3 (m, 4H), 2.3—1.7 (m, 7H), 2.11 (s, 3H, $CH_3$—CONH—)

Hydrochloride:

M.p. 245—247°C (decomp.) (colorless needles), recrystallized from ethanol)

IR$\nu_{max}^{nujol}$ ($cm^{-1}$): 3250—3050, 2455, 1655, 1140, 1040

Example 3

560 mg of 1-(4-acetamido-2-methoxyphenoxy)-3-(p-toluenesulfonyloxy)-n-propane, 500 mg of 4-(4-chlorophenyl)-4-hydroxy-piperidine, 250 mg of triethylamine and 20 ml of ethanol are treated in the same manner as described in Example 1-(4). The crude product obtained is recrystallized from ethanol. 525 mg of 1-(4-acetamido-2-methoxyphenoxy)-3-[4-(4-chlorophenyl)-4-hydroxy-piperidino]-n-propane are obtained as colorless granules. Yield: 86% M.p. 188—189°C

IR$\nu_{max}^{nujol}$ ($cm^{-1}$): 3280, 3130, 1655, 1135, 1125, 1040

NMR ($CDCl_3$+DMSO-$d_6$) $\delta$:

9.56 (s, 1H, —*NH*—, disappear with $D_2O$), 7.8—6.7 (m, 7H, aromatic), 4.06 (t, 2H, J=6.0 Hz, —$OCH_2CH_2$—), 3.80 (s, 3H, —$OCH_3$), 2.09 (s, 3H, $CH_3$CONH—)

Example 4

(1) 12.7 g of 4-acetamido-2-methoxy-phenol, 2.95 g of powdery sodium hydroxide, 11.7 g of 4-chloro-1-tetrahydropyran-2-yl-oxy)-n-butane and 180 ml of dimethylsulfoxide are treated in the same manner as described in Example 1-(1). The crude product thus obtained is recrystallized from a mixture of benzene and isopropyl ether. 18.08 g of 1-(4-acetamido-2-methoxyphenoxy)-4-tetrahydropyran-2-yl-oxy)-n-butane are obtained as colorless crystals. Yield: 76.5% M.p. 85—86°C.

(2) 18 g of 1-(4-acetamido-2-methoxyphenoxy)-4-(tetrahydropyran-2-yl-oxy)-n-butane, 60 ml of ethanol, 120 ml of water and 0.1 ml of concentrated hydrochloric acid are treated in the same manner as described in Example 1-(2). The crude product thus obtained is recrystallized from ethyl acetate. 13.3 g of 1-(4-acetamido-2-methoxyphenoxy)-4-hydroxy-n-butane are obtained as colorless needles. Yield: 98.6% M.p 107—108°C.

(3) 12.4 g of 1-(4-acetamido-2-methoxyphenoxy)-4-hydroxy-n-butane, 14.1 g of p-toluenesulfonyl chloride and 150 ml of pyridine are treated in the same manner as described in Example 1-(3). The crude product obtained is recrystallized from benzene. 11.37 g of 1-(4-acetamido-2-methoxyphenoxy)-4-(p-toluenesulfonyloxy)-n-butane are obtained as colorless needles. Yield: 57% M.p. 117—118°C.

(4) 1.5 g of 1-(4-acetamido-2-methoxyphenoxy)-4-(p-toluenesulfonyloxy)-n-butane, 710 mg of 4-phenylpiperidine, 1.5 ml of triethylamine and 15 ml of anhydrous dimethylformamide are treated in the same manner as described in Example 1-(4). The crude product thus obtained is recrystallized from a mixture of ethyl acetate and n-hexane. 1.3 g of 1-(4-acetamido-2-methoxyphenoxy)-4-(4-phenylpiperidino)-n-butane are obtained as colorless needles. Yield: 89% M.p. 117—118°C

IR$\nu_{max}^{nujol}$ (cm$^{-1}$): 3260, 3150, 1655, 1140, 1030

NMR ($CDCl_3$) $\delta$: 7.75 (broad s, 1H, —N*H*—), 7.44—6.6 (m, 8H, aromatic), 3.95 (broad t, 2H, J=5.4 Hz, —OC$H_2$C$H_2$—), 3.85 (s, 3H, —OC$H_3$), 3.3—2.8 (m, 3H), 2.7—1.0 (m, 12H), 2.14 (s, 3H, —C$H_3$CONH—)

Mass m/e: 396 ($M^+$), 2.6, 174 (base peak)

Hydrobromide:

M.p. 153—155°C (decomp.) (colorless needles), recrystallized from ethanol-ethyl acetate)

IR$\nu_{max}^{nujol}$ (cm$^{-1}$): 3500, 3430, 1655, 1150, 1020

Example 5

(1) A solution of 40 mg of sodium hydroxide in 40 ml of ethanol is added to a mixture of 1.95 g of 4-acetamido-2-methoxybenzylalcohol and 15 g of ethyl acrylate, and the mixture is stirred at room temperature for 20 hours. The reaction mixture is condensed under reduced pressure to remove excess ethyl acrylate. Chloroform is added to the residue obtained, and the chloroform solution is washed with water, dried and then evaporated under reduced pressure to remove solvent. The residue thus obtained is purified by silica gel chromatography (solvent: 1% methanol-chloroform). 1.02 g of 1-(4-acetamido-2-methoxybenzyloxy)-2-(ethoxycarbonyl)ethane are obtained. M.p. 86—87°C (recrystallized from isopropyl ether-benzene, colorless prisms).

(2) 1.56 g of lithium aluminium hydride are suspended in 250 ml of anhydrous tetrahydrofuran, and a solution of 5.87 g of 1-(4-acetamido-2-methoxybenzyloxy)-2-(ethoxycarbonyl)ethane in 150 ml of anhydrous tetrahydrofuran is added dropwise thereto at a temperature below 0°C under stirring. The mixture is further stirred at the same temperature for 3 hours. Then, excess lithium aluminium hydride is decomposed, and insoluble materials are removed by filtration. The filtrate is condensed to dryness under reduced pressure. 5.0 g of 1-(4-acetamido-2-methoxybenzyloxy)-3-hydroxy-n-propane are obtained as a viscous oil.

(3) 5.0 g of 1-(4-acetamido-2-methoxybenzyloxy)-3-hydroxy-n-propane are dissolved in 20 ml of pyridine, and a solution of 6.3 g of p-toluenesulfonyl chloride in 20 ml of pyridine is added dropwise thereto at a temperature below —10°C. The mixture is stirred at —10° to —5°C for 3 hours. The reaction mixture is poured into ice-water and extracted with methylene chloride. The extract is washed with 10% hydrochloric acid and water, dried and then evaporated under reduced pressure to remove solvent. The residue obtained is purified by silica gel chromatography (solvent: 1% methanol-chloroform). 7.22 g of 1-(4-acetamido-2-methoxybenzyloxy)-3-(p-toluenesulfonyloxy)-n-propane are obtained as a viscous oil. Yield: 89.5%.

(4) A solution of 2.0 g of 1-(4-acetamido-2-methoxybenzoyloxy)-3-(p-toluenesulfonyloxy)-n-propane and 1.75 g of 4-phenylpiperazine in 40 ml of ethanol is refluxed for 4 hours. After the reaction, said solution is evaporated under reduced pressure to remove solvent, and water is added to the residue. The aqueous mixture is extracted with methylene chloride, and the extract is washed with water, dried and then evaporated under reduced pressure to remove solvent. The residue thus obtained is purified by silica gel chromatography (solvent: 2% methanol-chloroform) and recrystallized from a mixture of benzene and isopropyl ether. 1.13 g of 1-(4-acetamido-2-methoxybenzyloxy)-3-(4-phenylpiperazino)-n-propane are obtained as colorless needles. Yield: 58% M.p. 104.5—105°C.

IR$\nu_{max}^{nujol}$ ($cm^{-1}$): 3250, 1660, 1610, 1130, 1100, 1030
NMR ($CDCl_3$) $\delta$ : 7.92 (broad s, 1H, —N*H*—), 7.5—6.6 (m, 8H, aromatic), 4.52 (s, 2H, Ar-$CH_2$—), 3.80 (s, 3H, —$OCH_3$), 3.59 (t, 2H, J=6.1 Hz, —$CH_2$—O—$CH_2$—), 3.4—3.0 (m, 4H), 2.8—2.3 (m, 6H), 2.3—1.6 (m, 2H), 2.13 (s, 3H, $CH_3$CONH—).

Example 6

(1) 1.0 g of 4-acetamidobenzylalcohol, 6.04 g of ethyl acrylate, 20 mg of sodium hydroxide and 2 ml of ethanol are treated in the same manner as described in Example 5-(1). 1.18 g of 1-(4-acetamidobenzyloxy)-2-(ethoxycarbonyl)ethane are obtained as a pale yellow oil. Yield: 63.5%.

(2) 9.9 g of 1-(4-acetamidobenzyloxy)-2-(ethoxycarbonyl)ethane, 1.7 g of lithium aluminium hydride and 400 ml of anhydrous ether are treated in the same manner as described in Example 5-(2). The crude product obtained is then recrystallized from a mixture of ethanol and isopropyl ether. 4.9 g of 1-(4-acetamidobenzoyloxy)-3-hydroxy-n-propane are obtained as colorless needles. Yield: 58.3% M.p. 89—90°C.

(3) 3.5 g of 1-(4-acetamidobenzoyloxy)-3-hydroxy-n-propane, 3.6 g of p-toluenesulfonyl chloride and 80 ml of pyridine are treated in the same manner as described in Example 5-(3). 5.2 g of 1-(4-acetamidobenzyloxy)-3-(p-toluenesulfonyloxy)-n-propane are obtained as an oil. Yield: 98%.

(4) 2.0 g of 1-(4-acetamidobenzyloxy)-3-(p-toluenesulfonyloxy)-n-propane, 1.9 g of 4-phenylpiperazine and 30 ml of ethanol are treated in the same manner as described in Example 5-(4). The crude product thus obtained is recrystallized from ether. 1.34 g of 1-(4-acetamidobenzoyloxy)-3-(4-phenylpiperazino)-n-propane are obtained as colorless needles. Yield: 70% M.p. 85—86.5°C.

IR$\nu_{max}^{nujol}$ ($cm^{-1}$): 3320, 1690, 1670, 1600
NMR ($CDCl_3$) $\delta$ : 7.75—6.9 (m, 10H, aromatic and —N*H*—), 4.48 (s, 2H, Ar-$CH_2$—O—), 3.54 (t, 2H, J=6.4 Hz, —$CH_2OCH_2CH_2$—), 3.3—3.1 (m, 4H), 2.7—2.3 (m, 6H), 2.13 (s, 3H, $CH_3$CONH—), 2.1—1.7 (m, 2H).

Example 7

(1) 0.2 ml of acetic acid is added to a mixture of 5.0 g of 4-phenylpiperazine and 17.7 g of ethyl 2-methylacrylate, and the mixture is stirred at 55° to 60°C for 4 hours. Benzene is added to the reaction mixture, and said mixture is extracted with 10% hydrochloric acid. The acidic extract is adjusted to pH 11 with potassium carbonate and again extracted with benzene. The benzene extract is washed with an aqueous saturated sodium chloride solution, dried and then evaporated under reduced pressure to remove solvent. The residue obtained is distilled under reduced pressure, whereby 3.16 g of ethyl 3-(4-phenylpiperazino)-2-methyl-n-propionate are obtained as a pale yellow oil. Yield: 37% B.p. 145—146°C/0.3 mmHg.

(2) 1.5 g of lithium aluminium hydride are suspended in 100 ml of anhydrous tetrahydrofuran, and a solution of 5.4 g of ethyl 3-(4-phenylpiperazino)-2-methyl-n-propionate in 50 ml of anhydrous tetrahydrofuran is added dropwise thereto at a temperature below 5°C. The mixture is stirred at the same temperature for 2 hours. Then, excess lithium aluminium hydride is decomposed, and insoluble materials are removed by filtration. The filtrate is condensed to dryness under reduced pressure. The residue obtained is recrystallized from isopropyl ether. 3.56 g of 3-(4-phenylpiperazino)-2-methyl-n-propanol are obtained as colorless needles. Yield: 78% M.p. 77—78°C.

(3) 3.0 g of 3-(4-phenylpiperazino)-2-methyl-n-propanol and 1.95 g of triethylamine are dissolved in 25 ml of methylene chloride, and a solution of 3.73 g of p-toluenesulfonyl chloride in 15 ml of methylene chloride is added dropwise thereto. The mixture is refluxed for 80 hours. After cooling the reaction mixture, said mixture is washed with water, dried and then evaporated under reduced pressure to remove solvent. The residue obtained is purified by silica gel chromatography (solvent: 1% methanol-chloroform) and recrystallized from n-hexane. 1.55 g of 3-(4-phenylpiperazino)-2-methyl-n-propyl chloride are obtained as colorless needles. Yield: 52% M.p. 65—66°C.

(4) 1.23 g of 4-acetamido-2-methoxyphenol are dissolved in 20 ml of anhydrous dimethylsulfoxide, and 286 mg of powdery sodium hydroxide are added thereto in nitrogen atmosphere. The mixture is stirred at 50°C for 2 hours. Then, a solution of 1.42 g of 3-(4-phenylpiperazino)-2-methyl-n-propyl chloride in 10 ml of anhydrous dimethylsulfoxide is added dropwise to the mixture at 20°C, and said mixture is stirred for 1.5 hours under heating at 100°C. After cooling, the reaction mixture is poured into ice-water and extracted with chloroform. The extract is washed with water, dried and evaporated under reduced pressure to remove solvent. The crude product (2.2 g) thus obtained is dissolved in ethyl acetate under heating, treated with active carbon and then recrystallized from ethyl acetate. 1.8 g of 1-(4-acetamido-2-methoxyphenoxy)-3-(4-phenylpiperazino)-2-methyl-n-propane are obtained as colorless needles. Yield: 80.5% M.p. 155—156°C.

IR$\nu_{max}^{nujol}$ ($cm^{-1}$): 3325, 1655, 1145, 1020
NMR ($CDCl_3$) $\delta$ : 7.60 (broad s, 1H, —N*H*—), 7.4—6.6 (m, 8H, aromatic), 3.96 (m, 2H,

—O—$CH_2$—CH—),
|

3.77 (s, 3H, —$OCH_3$), 3.4—3.0 (m, 4H) 2.8—2.0 (m, 7H), 2.08 (s, 3H, $CH_3$CONH—), 1.08 (d, 3H, J=5.5 Hz, >CH—$CH_3$)

Mass m/e: 397 ($M^+$) 217, 175 (base peak)

Hydrochloride:

M.p. 248—249°C (decomp.) (colorless prisms, recrystallized from methanol)

IR$\nu_{max}^{nujol}$ ($cm^{-1}$): 3250—3050, 2700—2300, 1665, 1605, 1030

Example 8

(1) 5 g of 4-phenylpiperazine, 17.7 g of ethyl crotonate and 0.2 ml of acetic acid are treated in the same manner as described in Example 7-(1). 4.0 g of ethyl 3-(4-phenylpiperazino)-3-methyl-n-propionate are obtained as a pale yellow oil. Yield: 46.8% B.p. 159—160°C/0.4 mmHg.

(2) 4.0 g of ethyl 3-(4-phenylpiperazino)-3-methyl-n-propionate, 1.1 g of lithium aluminium hydride and 150 ml of anhydrous tetrahydrofuran are treated in the same manner as described in Example 7-(2). The crude product obtained is recrystallized from isopropyl ether. 2.86 g of 3-(4-phenylpiperazino)-3-methyl-n-propanol are obtained as colorless needles. Yield: 84% M.p. 86—87°C.

(3) 3.0 g of 3-(4-phenylpiperazino)-3-methyl-n-propanol, 1.95 g of triethylamine, 3.73 g of p-toluenesulfonyl chloride and 40 ml of methylene chloride are treated in the same manner as described in Example 7-(3). 2.19 g of 3-(4-phenylpiperazino)-1-methyl-n-propyl chloride (pale yellow oil) and 50 mg of 3-(4-phenylpiperazino)-3-methyl-n-propyl chloride (oil) are obtained respectively.

(4) 1.85 g of 4-acetoamido-2-methoxyphenol, 430 mg of powdery sodium hydroxide, 2.15 g of 3-(4-phenylpiperazino)-1-methyl-n-propyl chloride and 40 ml of anhydrous dimethylsulfoxide are treated in the same manner as described in Example 7-(4). The crude product thus obtained is treated with hydrochloric acid. Fractional recrystallization of the thus-obtained crystalline precipitates from methanol gives 1.13 g of 1-(4-acetamido-2-methoxyphenoxy)-3-(4-phenylpiperazino)-1-methyl-n-propane hydrochloride (Compound A) and 560 mg of 1-(4-acetamido-2-methoxyphenoxy)-3-(4-phenylpiperazino)-3-methyl-n-propane hydrochloride (Compound B), respectively.

*Compound A:*

Colorless prisms

M.p. 218—222°C

IR$\nu_{max}^{nujol}$ ($cm^{-1}$): 3250—3000, 2750—2300, 1670, 1145, 1040

*Compound A (free base):*

M.p. 106—108°C (colorless needles, recrystallized from benzene-isopropyl ether)

IR$\nu_{max}^{nujol}$ ($cm^{-1}$): 3300—3200, 1650, 1600, 1140, 1040

NMR ($CDCl_3$) $\delta$ : 7.75 (broad, 1H, —$NH$—, disappear with $D_2O$), 7.6—6.6 (m, 8H, aromatic), 4.3 (m, 1H,

—O$CH$—),
|

3.76 (s, 3H, —$OCH_3$), 3.4—3.0 (m, 4H), 2.8—2.3 (m, 6H), 2.3—1.8 (m, 2H), 2.10 (s, 3H, $CH_3$CONH—), 1.30 (d, 3H, J=6.3 Hz, —O—CH($CH_3$)—)

Mass m/e: 397 ($M^+$), 217, 175 (base peak)

*Compound B:*

Colorless prisms

M.p. 262—264°C

IR$\nu_{max}^{nujol}$ ($cm^{-1}$): 3300—3000, 2700—2300, 1675, 1140, 1040

*Compound B (free base):*

M.p. 120—121°C (colorless needles, recrystallized from benzene)

IR$\nu_{max}^{nujol}$ ($cm^{-1}$): 3250, 1650, 1140, 1040

NMR ($CDCl_3$) $\delta$ : 7.87 (broad, 1H, —$NH$—, disappear with $D_2O$), 7.4—6.6 (m, 8H, aromatic), 4.04 (t, 2H, J=7.0 Hz, —O$CH_2$$CH_2$—), 3.77 (s, 3H, —$OCH_3$), 3.4—2.4 (m, 9H), 2.3—1.6 (m, 2H), 2.08 (s, 3H, $CH_3$CO—NH—), 1.05 (d, 3H, J=6.7 Hz, —($CH_3$)CH—N<)

Mass m/e: 397 ($M^+$), 217, 180 (base peak).

**Claims**

1. A compound of the formula:

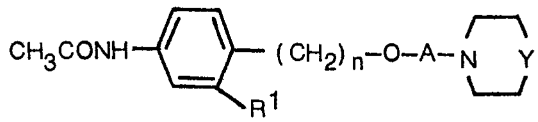

wherein $R^1$ is methoxy, n is zero, A is alkylene of 2 to 4 carbon atoms and Y is a group of the formula:

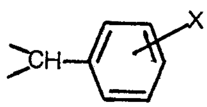 or 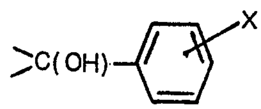

wherein X is hydrogen or halogen; or

$R^1$ is hydrogen or methoxy, n is an integer of 1, A is alkylene of 2 to 3 carbon atoms and Y is a group of the formula:

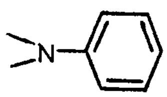 ;

or

$R^1$ is methoxy, n is zero, Y is a group of the formula:

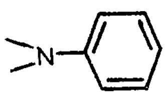 ,

and A is a branched alkylene of the formula:

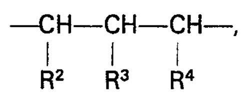

wherein either one of $R^2$, $R^3$ and $R^4$ is methyl and the remaining two groups of $R^2$, $R^3$ and $R^4$ are hydrogen,
or a pharmaceutically acceptable acid addition salt thereof.

2. The compound of Claim 1, in which $R^1$ is methoxy, n is zero, A is ethylene or trimethylene, Y is a group of the formula:

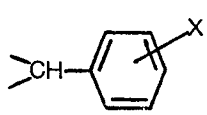 or 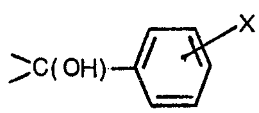

and X is hydrogen or halogen; or

$R^1$ is hydrogen or methoxy, n is an integer of 1, A is ethylene or trimethylene, and Y is a group of the formula:

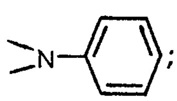 ;

or

$R^1$ is methoxy, n is zero, A is a group of the formula:

$$-CH_2CH(CH_3)CH_2-,\ -CH_2CH_2CH(CH_3)-\ \text{or}\ -CH(CH_3)CH_2CH_2-,$$

and Y is a group of the formula:

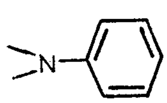

3. The compound of Claim 2, in which $R^1$ is methoxy, n is zero, A is trimethylene and Y is a group of the formula:

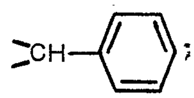

or

$R^1$ is hydrogen or methoxy, n is an integer of 1, A is trimethylene and Y is a group of the formula:

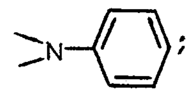

or

$R^1$ is methoxy, n is zero, A is a group of the formula:

$$-CH_2CH(CH_3)CH- \text{ or } -CH_2CH_2CH(CH_3)-,$$

and Y is a group of the formula:

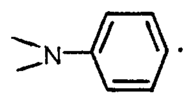

4. The compound of Claim 3, in which $R^1$ is hydrogen or methoxy, n is an integer of 1, A is trimethylene and Y is a group of the formula:

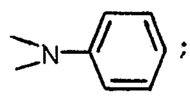

or

$R^1$ is methoxy, n is zero, A is a group of the formula:

$$-CH_2CH(CH_3)CH_2-,$$

and Y is a group of the formula:

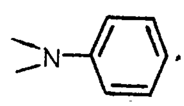

5. The compound of Claim 4 which is 1-(4-acetamidobenzyloxy)-3-(4-phenylpiperazino)-n-propane or a pharmaceutically acceptable acid addition salt thereof.

6. The compound of Claim 4 which is 1-(4-acetamido-2-methoxyphenoxy)-3-(4-phenylpiperazino)-2-methyl-n-propane or a pharmaceutically acceptable acid addition salt thereof.

7. The compound of Claim 4 which is 1-(4-acetamido-2-methoxybenzyloxy)-3-(4-phenylpiperazino)-n-propane or a pharmaceutically acceptable acid addition salt thereof.

8. A process for preparing a compound of the formula:

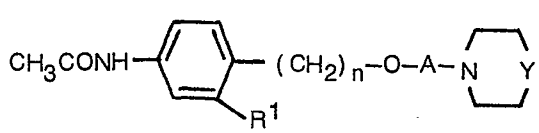

wherein $R^1$ is methoxy, n is zero, A is alkylene of 2 to 4 carbon atoms, Y is a group of the formula:

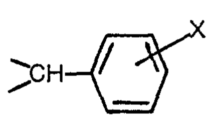

or

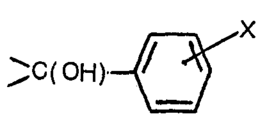

and X is hydrogen or halogen; or

$R^1$ is hydrogen or methoxy, n is an integer of 1, A is alkylene of 2 to 3 carbon atoms and Y is a group of the formula:

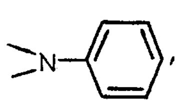

or a pharmaceutically acceptable acid addition salt thereof, which comprises condensing a compound of the formula:

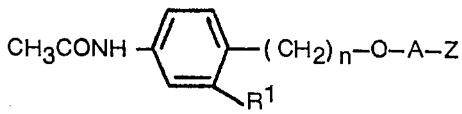

wherein Z is a reactive group or atom and $R^1$, A and n are the same as defined above, with a compound of the formula:

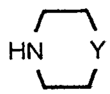

wherein Y is the same as defined above, and if required, further converting the product into a pharmaceutically acceptable acid addition salt thereof.

9. The process according to Claim 8, wherein the group Z is alkylsulfonyloxy, arylsulfonyloxy or halogen, and the condensation reaction is carried out at 20° to 100°C in an inert solvent.

10. A process for preparing a compound of the formula:

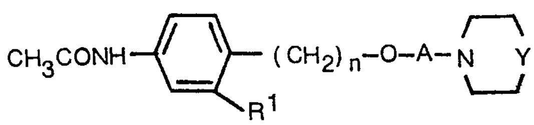

wherein $R^1$ is methoxy, n is zero, Y is a group of the formula:

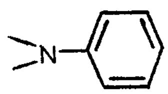

A is a branched alkylene of the formula:

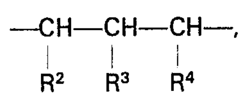

and either one of $R^2$, $R^3$, and $R^4$ is methyl and the remaining two groups of $R^2$, $R^3$ and $R^4$ are hydrogen, or a pharmaceutically acceptable acid addition salt thereof, which comprises condensing a compound of the formula:

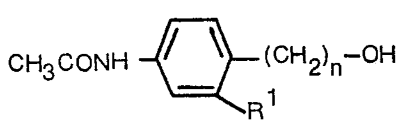

wherein $R^1$ and n are the same as defined above, with a compound of the formula:

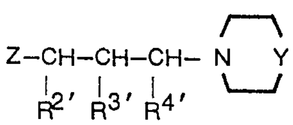

wherein either one of $R^{2'}$, $R^{3'}$ and $R^{4'}$ is methyl and the remaining two groups of $R^{2'}$, $R^{3'}$ and $R^{4'}$ are hydrogen, Z is a reactive group or atom, and if required, further converting the product into a pharmaceutically acceptable acid addition salt thereof.

11. The process according to Claim 10, wherein the group Z is alkylsulfonyloxy, arylsulfonyloxy or halogen, and the condensation reaction is carried out in the presence of an acid acceptor at 20°C to 100°C in an inert solvent.

12. A pharmaceutical composition characterized by comprising a compound of Claims 1 to 7 together with a pharmaceutically acceptable carrier therefore.

**Revendications**

1. Composé caractérisé en ce qu'il est représenté par la formule générale (I) ci-après:

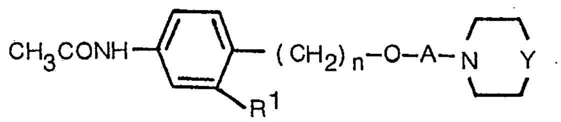

dans laquelle $R^1$ est un radical méthoxy, n est égal à zéro, A est un radical alkylène de 2 à 4 atomes de carbone et Y est un groupe de formule:

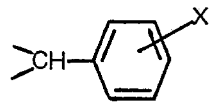 ou 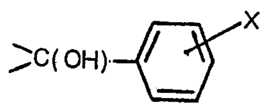

où X est un atome d'hydrogène ou d'halogène; ou bien $R^1$ est un atome d'hydrogène ou un radical méthoxy, n est égal à 1, A est un radical alkylène de 2 à 3 atomes de carbone et Y est un groupe un groupe de formule:

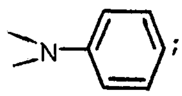 ;

ou bien $R^1$ est un radical méthoxy, n est égal à zéro, Y est un groupe de formule:

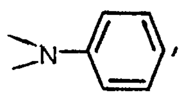 ,

et A est un radical alkylène ramifié de formule:

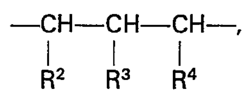 ,

dans lequel l'un des groupes $R^2$, $R^3$ et $R^4$ est un radical méthyle et les deux autres sont chacun un atome d'hydrogène, ou un sel d'addition acide acceptable du point de vue pharmaceutique, de ce composé.

2. Composé selon la revendication 1, caractérisé en ce que $R^1$ est un radical méthoxy, n est égal à zéro, A est un groupe éthylène ou triméthylène, Y est un groupe de formule:

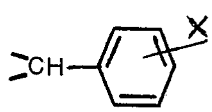 ou 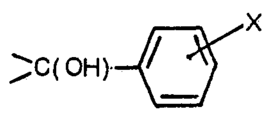

et X est un atome d'hydrogène ou d'halogène; ou bien $R^1$ est un atome d'hydrogène ou un radical méthoxy, n est égal à 1, A est un groupe éthylène ou triméthylène et Y est un groupe de formule:

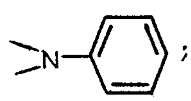 ;

ou bien $R^1$ est un radical méthoxy, n est égal à zéro, A est un groupe représenté par la formule:

$$-CH_2CH(CH_3)CH_2-,\ CH_2CH_2CH(CH_3)-\ \text{ou}\ -CH(CH_3)CH_2CH_2-$$

et Y est un groupe de formule:

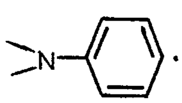 .

3. Composé selon la revendication 2, caractérisé en ce que $R^1$ est un groupe méthoxy, n est égal à zéro, A est un radical triméthylène et Y est un groupe de formule:

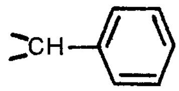 ;

ou bien, $R^1$ est un atome d'hydrogène ou un groupe méthoxy, n est égal à 1, A est un groupe triméthylène et Y est un groupe de formule:

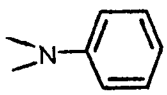 ;

ou bien $R^1$ est un groupe méthoxy, n est égal à zéro, A est un groupe de formule:

$$-CH_2CH(CH_3)CH_2-, \text{ ou } -CH_2CH_2CH(CH_3)-,$$

et Y est un groupe de formule:

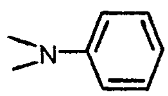 .

4. Composé selon la revendication 3, caractérisé en ce que $R^1$ est un atome d'hydrogène ou un radical méthoxy, n est égal à 1, A est un groupe triméthylène et Y est un groupe de formule:

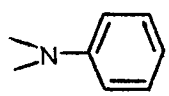 ;

ou bien, $R^1$ est un radical méthoxy, n est égal à zéro, A est un groupe de formule:

$$-CH_2CH(CH_3)CH_2-$$

et Y est un groupe de formule:

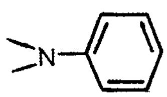 .

5. Composé selon la revendication 4, caractérisé en ce qu'il s'agit du 1-(4-acétamido-benzyloxy)-3-(4-phénylpipérazino)-n-propane ou d'un sel d'addition acide acceptable du point de vue pharmaceutique de celui-ci.

6. Composé selon la revendication 4, caractérisé en ce qu'il s'agit du 1-(4-acétamido-2-méthoxy-phénoxy)-3-(4-phénylpipérazino)-2-méthyl-n-propane ou d'un sel d'addition acide acceptable du point de vue pharmaceutique de celui-ci.

7. Composé selon la revendication 4, caractérisé en ce qu'il s'agit du 1-(4-acétamido-2-méthoxy-benzyloxy)-3-(4-phénylpipérazino)-n-propane ou d'un sel d'addition acide acceptable du point de vue pharmaceutique de celui-ci.

8. Procédé pour préparer un composé représenté par la formule générale suivante:

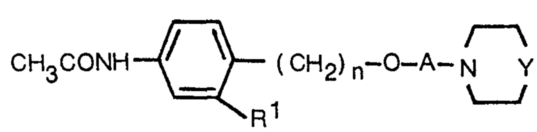

dans laquelle $R^1$ est un grope méthoxy, n est égal à zéro, A est un radical alkylène de 2 à 4 atomes de carbone, Y est un groupe de formule:

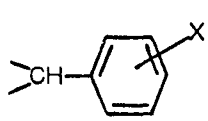 ou 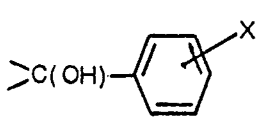

où X est un atome d'hydrogène ou d'halogène; ou bien $R^1$ est un atome d'hydrogène ou un radical méthoxy, n est égal à 1, A est un groupe alkylène de 2 à 3 atomes de carbone et Y est un groupe de formule:

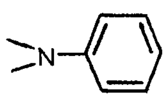 ,

ou l'un de ses sels d'addition acides acceptables du point de vue pharmaceutique, caractérisé en ce qu'on condense un composé représenté par la formule générale ci-après:

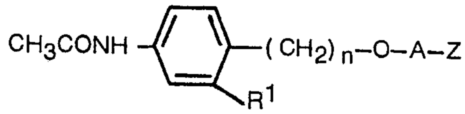

dans laquelle Z est un atome ou un groupe réactif et $R^1$, A et n sont tels que définis plus haut, avec un composé de formule générale ci-après:

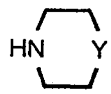

où Y est tel qui défini plus haut, et éventuellement on convertit ensuite le produit obtenu en un sel d'addition acide acceptable du point de vue pharmaceutique de celui-ci.

9. Procédé selon la revendication 8, caractérisé en ce que le groupe Z est un radical alkylsulfonyloxy, arylsulfonyloxy ou un atome d'halogène et que la réaction de condensation est réalisée entre 20 et 100°C dans un solvant inerte.

10. Procédé pour préparer un composé représenté par la formule générale ci-après:

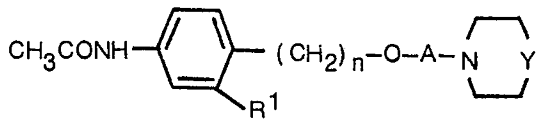

dans laquelle $R^1$ est un radical méthoxy, n est égal à zéro, Y est un groupe de formule:

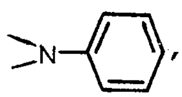

A est un radical alkylène ramifié de formule:

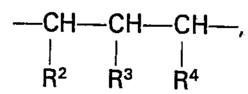

dans laquelle l'un des radicaux $R^2$, $R^3$ et $R^4$ est un groupe méthyle et les autres sont chacun un atome d'hydrogène, ou l'un de ses sels d'addition acides acceptables du point de vue pharmaceutique, caractérisé en ce qu'on condense un composé représenté par la formule générale ci-après:

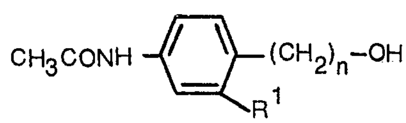

où $R^1$ et n sont tels que définis plus haut, avec un composé représenté par la formule générale ci-après:

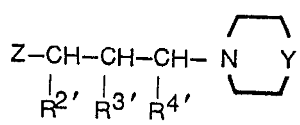

dans laquelle l'un des radicaux $R^{2'}$, $R^{3'}$ et $R^{4'}$ est un groupe méthyle et les autres sont chacun un atome d'hydrogène, Z est un atome ou un groupe réactif et éventuellement on convertit encore le produit obtenu, en un sel d'addition acide acceptable du point de vue pharmaceutique de celui-ci.

11. Procédé selon la revendication 10, caractérisé en ce que le groupe Z est un radical alkylsulfonyloxy, arylsulfonyloxy ou un atome d'halogène et que la réaction de condensation est effectuée en présence d'un accepteur d'acide, entre 20 et 100°C dans un solvant inerte.

12. Composition pharmaceutique caractérisée en ce qu'elle comprend un composé selon les revendications 1 à 7, ainsi qu'un support pour celui-ci, acceptable du point de vue pharmaceutique.

**Patentansprüche**

1. Verbindung der Formel:

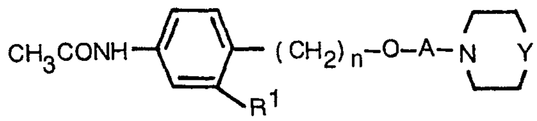

worin $R^1$ für Methoxy steht, n O ist, A für Alkylen mit 2 bis 4 Kohlenstoffatomen steht und Y für eine Gruppe der Formel

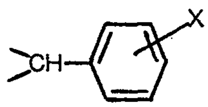 oder 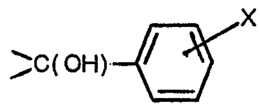

steht, wobei X für Wasserstoff oder Halogen steht, oder worin $R^1$ für Wasserstoff oder Methoxy steht, n eine ganze Zahl von 1 ist, A für Alkylen mit 2 bis 3 Kohlenstoffatomen steht und Y eine Gruppe der Formel

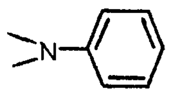

ist oder worin $R^1$ für Methoxy steht, n O ist, Y eine Gruppe der Formel

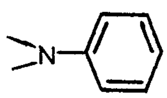

ist und A verzweigtes Alkylen der Formel

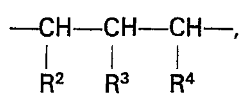

ist, wobei eine der Gruppen $R^2$, $R^3$ und $R^4$ Methyl ist und die restlichen zwei Gruppen $R^2$, $R^3$ und $R^4$ Wasserstoff sind, oder ein pharmazeutisch annehmbares Säureadditionssalz davon.

2. Verbindung nach Anspruch 1, dadurch gekennzeichnet, daß $R^1$ für Methoxy steht, n O ist, A für Ethylen oder Trimethylen steht, Y für eine Gruppe der Formel

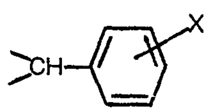 oder 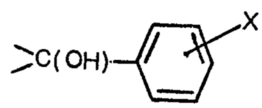

steht und X für Wasserstoff oder Halogen steht, oder daß $R^1$ für Wasserstoff oder Methoxy steht, n eine ganze Zahl von 1 ist, A für Ethylen oder Trimethylen steht und Y eine Gruppe der Formel

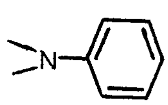

ist, oder daß $R^1$ für Methoxy steht, n O ist, A für eine Gruppe der Formel

$$-CH_2CH(CH_3)CH_2-,\ -CH_2CH_2CH(CH_3)-\text{ oder }-CH(CH_3)CH_2CH_2-$$

steht und Y für eine Gruppe der Formel

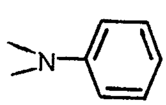

steht.

3. Verbindung nach Anspruch 2, dadurch gekennzeichnet, daß $R^1$ für Methoxy steht, n O ist, A für Trimethylen steht und Y eine Gruppe der Formel

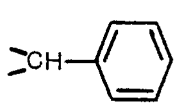

bedeutet oder daß R[1] für Wasserstoff oder Methoxy steht, n eine ganze Zahl von 1 ist, A für Trimethylen steht und Y für eine Gruppe der Formel

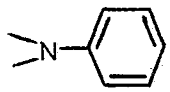

steht, oder daß R[1] für Methoxy steht, n O ist, A für eine Gruppe der Formel

$$-CH_2CH(CH_3)CH_2-\text{, oder }-CH_2CH_2CH(CH_3)-$$

steht und Y für eine Gruppe der Formel

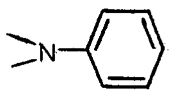

steht.

4. Verbindung nach Anspruch 3, dadurch gekennzeichnet, daß R[1] für Wasserstoff oder Methoxy steht, n eine ganze Zahl von 1 ist, A für Trimethylen steht und Y für eine Gruppe der Formel

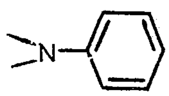

steht, oder daß R[1] für Methoxy steht, n O ist, A für eine Gruppe der Formel

$$-CH_2CH(CH_3)CH_2-$$

steht und Y für eine Gruppe der Formel

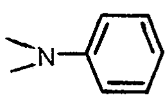

steht.

5. Verbindung nach Anspruch 4, nämlich 1-(4-Acetamidobenzyloxy)-3-(4-phenylpiperazino)-n-propan, oder ein pharmazeutisch annehmbares Säureadditionssalz davon.

6. Verbindung nach Anspruch 4, nämlich 1-(4-Acetamido-2-methoxyphenoxy)-3-(4-phenylpiperazino)-2-methyl-n-propan, oder ein pharmazeutisch annehmbares Säureadditionssalz davon.

7. Verbindung nach Anspruch 4, nämlich 1-(4-Acetamido-2-methoxybenzyloxy)-3-(4-phenylpiperazino)-n-propan, oder ein pharmazeutisch annehmbares Säureadditionssalz davon.

8. Verfahren zur Herstellung einer Verbindung der Formel:

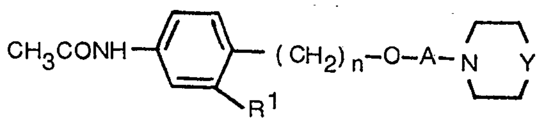

worin R[1] für Methoxy steht, n O ist, A für Alkylen mit 2 bis 4 Kohlenstoffatomen steht, Y für eine Gruppe der Formel

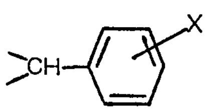

oder

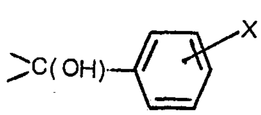

steht und X für Wasserstoff oder Halogen steht, oder worin R[1] für Wasserstoff oder Methoxy steht, n eine ganze Zahl von 1 ist, A für Alkylen mit 2 bis 3 Kohlenstoffatomen steht und Y für eine Gruppe der Formel

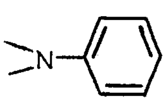

steht, oder eines pharmazeutisch annehmbaren Säureadditionssalzes davon, dadurch gekennzeichnet, daß man eine Verbindung der Formel:

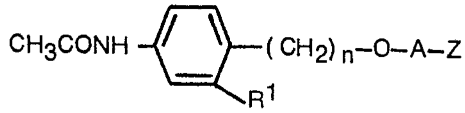

worin Z für eine reaktive Gruppe oder ein reaktives Atom steht und $R^1$, A und n die oben angegebenen Bedeutungen haben, mit einer Verbindung der Formel:

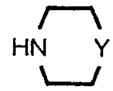

worin Y die oben angegebene Bedeutung hat, kondensiert und erforderlichenfalls das Produkt in ein pharmazeutisch annehmbares Säureadditionssalz davon weiter umwandelt.

9. Verfahren nach Anspruch 8, dadurch gekennzeichnet, daß die Gruppe Z Alkylsulfonyloxy, Arylsulfonyloxy oder Halogen ist und daß man die Kondensationsreaktion bei 20 bis 100°C in einem inerten Lösungsmittel durchführt.

10. Verfahren zur Herstellung einer Verbindung der Formel:

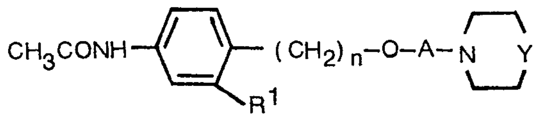

worin $R^1$ für Methoxy steht, n O ist, Y für eine Gruppe der Formel

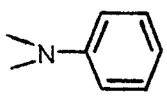

steht, A für verzweigtes Alkylen der Formel

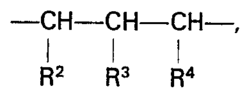

steht und eine der Gruppen $R^2$, $R^3$ und $R^4$ Methyl ist und die restlichen zwei Gruppen $R^2$, $R^3$ und $R^4$ Wasserstoff sind, oder eines pharmazeutisch annehmbaren Säureadditionssalzes davon, dadurch gekennzeichnet, daß man eine Verbindung der Formel:

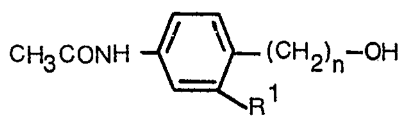

worin $R^1$ und n die oben angegebenen Bedeutungen haben, mit einer Verbindung der Formel:

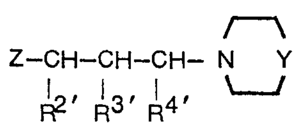

worin eine der Gruppen $R^{2'}$, $R^{3'}$ und $R^{4'}$ Methyl ist und die restlichen zwei Gruppen $R^{2'}$, $R^{3'}$ und $R^{4'}$ Wasserstoff sind, Z eine reaktive Gruppe oder ein reaktives Atom ist, kondensiert und erforderlichenfalls das Produkt zu einem pharmazeutisch annehmbaren Säureadditionssalz davon weiter umsetzt.

11. Verfahren nach Anspruch 10, dadurch gekennzeichnet, daß die Gruppe Z Alkylsulfonyloxy, Arylsulfonyloxy oder Halogen ist und daß man die Kondensationsreaktion in Gegenwart eines Säureakzeptors bei 20 bis 100°C in einem inerten Lösungsmittel durchführt.

12. Arzneimittel, dadurch gekennzeichnet, daß es eine Verbindung nach den Ansprüchen 1 bis 7 zusammen mit einem pharmazeutisch annehmbaren Träger hierfür enthält.